(19) Europäisches Patentamt
European Patent Office
Office européen des brevets

(11) Publication number: 0 073 100
B1

# (12) EUROPEAN PATENT SPECIFICATION

(45) Date of publication of patent specification: 02.10.85

(21) Application number: 82303858.3

(22) Date of filing: 22.07.82

(51) Int. Cl.[4]: C 07 D 487/04, A 61 K 31/40 // C07D205/08, C07F9/65, C07C79/43, C07C101/28, C07C93/14 ,(C07D487/04, 209:00, 205:00)

## (54) Beta-lactam compounds, their preparation and their use.

(30) Priority: 13.08.81 GB 8124817

(43) Date of publication of application:
02.03.83 Bulletin 83/09

(45) Publication of the grant of the patent:
02.10.85 Bulletin 85/40

(84) Designated Contracting States:
CH DE FR GB IT LI NL

(56) References cited:
EP-A-0 034 443
US-A-4 218 459
US-A-4 218 463

(73) Proprietor: BEECHAM GROUP PLC
Beecham House Great West Road
Brentford Middlesex TW8 9BD (GB)

(72) Inventor: Pearson, Michael John
33 Greenfields Road
Roffey Horsham Sussex (GB)
Inventor: Branch, Clive Leslie
18 Overdale
Dorking Surrey (GB)

(74) Representative: Hesketh, Alan, Dr. et al
European Patent Attorney Beecham
Pharmaceuticals Great Burgh Yew Tree Bottom
Road
Epsom, Surrey, KT18 5XQ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel β-lactam anti-bacterial agents, their preparation and their use, and in particular to 7-oxo-1-azabicyclo[3.2.0]heptanes.

The present invention provides a compound of formula (I):

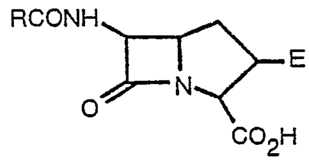

(I)

or a pharmaceutically acceptable salt or cleavable ester thereof, wherein RCONH— represents an organic acylamino group found in antibacterially effective penicillins and cephalosposins and E is a cyano group or a carboxy group or a pharmaceutically acceptable salt or ester thereof of sub-formula (L) or (M):

$$CO_2B_1 \quad (L)$$

$$CO_2CHB_2B_3 \quad (M)$$

wherein $B_1$ is an alkyl group of 1 to 6 carbon atoms optionally substituted by an alkoxy or alkanoyloxy group of 1 to 7 carbon atoms, $B_2$ is an alkenyl or alkynyl group of up to 5 carbon atoms or is a phenyl group optionally substituted by one, two or three atoms or groups selected from fluorine, chlorine, bromine, nitro or alkyl or alkoxy of up to 4 carbon atoms; and $B_3$ is a hydrogen atom, an alkyl group of up to 4 carbon atoms or is a phenyl group optionally substituted by one, two or three atoms or groups selected from fluorine, chlorine, bromine, nitro or alkyl or alkoxy of up to 4 carbon atoms.

Suitably E is carboxy or $C_{1-6}$ alkoxycarbonyl.

Suitably $B_1$ is an alkyl group of 1 to 6 carbon atoms.

More suitably $B_1$ is a methyl, ethyl, propyl, isopropyl, butyl, isobutyl, sec-butyl, tert-butyl, pentyl, neopentyl, isopentyl or hexyl group.

In particular $B_1$ is a methyl, ethyl or propyl group.

A preferred value for $B_1$ is a methyl group.

More suitably $B_2$ is a hydrogen atom and $B_3$ is a phenyl group optionally substituted by a fluorine, chlorine, bromine, nitro or alkyl or alkoxy of up to 4 carbon atoms.

In particular $CHB_2B_3$ is a benzyl, methoxybenzyl ethoxybenzyl, nitrobenzyl or chlorobenzyl group.

A preferred value for E is a cyano group.

Suitable organic acylamino groups RCONH found in antibacterially effective penicillins and cephalosporins include those of sub-formulae (a), (b), (c) and (d):

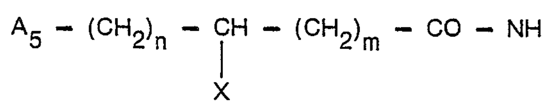

(a)

$$A_6 - CO - NH \quad (b)$$

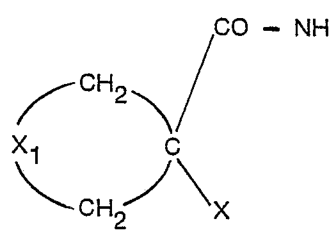

(c)

$$A_6 - X_2 - (CH_2)_n - CO - NH \quad (d)$$

wherein n is 0, 1 or 2; m is 0, 1 or 2; $A_5$ is an alkyl group of 1 to 6 carbon atoms, a cycloalkyl group of 3 to 6 carbon atoms, cyclohexenyl, cyclohexadienyl, phenyl, hydroxyphenyl, thienyl or pyridyl group; X is a hydrogen, bromine or chlorine atoms, a carboxylic acid, carboxylate ester, tetrazolyl, azido, hydroxy, acyloxy, amino, acylamino, heterocyclylamino, ureido, guanidino or acylureido group; $A_6$ is an aromatic

group such as a phenyl, 2,6-dimethoxyphenyl, 2-alkoxy-1-naphthyl, 3-arylisoxazolyl, isothiazolyl or 3-aryl-5-methylisoxazolyl group; $X_1$ is a $CH_2OCH_2$, $CH_2SCH_2$ or $(CH_2)_n$ group; and $X_2$ is an oxygen or sulphur atom. For example, suitable groups RCONH include phenylacetamido, p - hydroxyphenylacetamido, o - hydroxyphenylacetamido, m - hydroxyphenylacetamido, α - chlorophenylacetamido, α - bromophenylacetamido, α - carboxyphenylacetamido and esters thereof such as the methylphenyl, indanyl and phenyl esters, α - azidophenylacetamido, α - aminophenylacetamido, α - hydroxyphenylacetamido, α - ureidophenylacetamido, α - guanidino-phenylacetamido, α - (acetylureido)phenylacetamido, α - acetoxyphenylacetamido, α - tetrazolylphenylacetamido, acetamido, chloroacetamido, bromoacetamido, propionamido, pyridylacetamido, 2 - thienylacetamido, 3-thienylacetamido, 2 - thienylpropionamido, 3 - thienylpropionamido, α - chloro (p - hydroxyphenyl)acetamido, α - bromo(p - hydroxyphenyl) acetamido, α - carboxy(p - hydroxyphenyl) acetamido and esters thereof such as the methylphenyl, indanyl and phenyl esters α - amino(p - hydroxyphenyl)acetamido, α - hydroxy(p - hydroxyphenyl) acetamido, α - acetoxy(p - hydroxyphenyl)acetamido, α - ureido(p - hydroxyphenyl)acetamido, α-guanidino (p-hydroxyphenyl)acetamido, α-acetylureido(p-hydroxyphenyl)acetamido, phenoxyacetamido, o-hydroxyphenoxyacetamido, m-hydroxyphenoxyacetamido, p-hydroxyphenoxyacetamido, methoxyacetamido. ethoxyacetamido, α-amino(p-hydroxy)phenoxyacetamido, α-aminophenoxyacetamido, α-acetylphenoxyacetamido, α-acetyl(p-hydroxy)phenylacetamido, α-hydroxyphenoxyacetamido, α-hydroxy(p-hydroxy)-phenylacetamido, α-carboxyphenoxyacetamido and esters thereof such as the methylphenyl, indanyl and phenyl esters, α-carboxy(p-hydroxy) phenoxyacetamido and esters thereof such as the methylphenyl, indanyl and phenyl esters, phenoxypropionamido, phenoxybutyramido, benzamido, 2,6-dimethoxybenzamido, 2-ethoxy-1-naphthamido, 3-methoxy-1-naphthamido, , 2-propoxy-1-naphthamido, 3-phenyl-5-methyl-4-isoxazolylcarboxamido, 3-o-chlorophenyl-5-methyl-4-isoxazolylcarboxamido, 3-o,o-dichlorophenyl-5-methyl-4-isoxazolylcarboxamido, isothiazolylcarboxamido, 3-o,o-fluorochlorophenyl-5-methyl-4-isoxazolylcarboxamido, 3-phenyl-4-isoxazolylcarboxamido, 3-o-chlorophenyl-4-isoxazolyl-carboxamido, 3-o,o-dichlorophenyl-4-isoxazolylcarboxamido, 3-o,o-fluorochlorophenyl-4-isoxazolylcarboxamido, 1-aminocyclohexyl-1-carboxamido, phenylthioacetamido, phenylthiopropionamido, p-hydroxyphenylthioacetamido, and the like.

More suitably groups R CONH include those of the sub-formulae (a) and (f):

$$R^2\text{—}\underset{\displaystyle R^3}{\underset{|}{CH}}\text{—CO—NH} \qquad (e)$$

$$R^4\text{—}\underset{\displaystyle R^5}{\underset{|}{CH}}\text{—CO—NH} \qquad (f)$$

wherein $R^2$ is a phenyl, thienyl or phenoxy group; $R^3$ is a hydrogen atom or methyl group; $R^4$ is a phenyl, p-hydroxyphenyl, cyclohexadienyl, or a 5- or 6-membered heteroaryl or heterocyclyl group containing up to 3 hetero atoms selected from sulphur, oxygen and nitrogen, said group being optionally substituted by one, two or three substituents selected from hydroxy, amino, halo and $C_{1-6}$ alkoxy; $R^5$ is a hydroxy, amino or carboxy or phenyl, methylphenyl, indanyl, or $C_{1-6}$ alkyl ester thereof, or ureido of sub formula —$NHCONR^7R^8$.

Examples of suitable groups $R^4$ include thienyl, pyridyl and aminothiazolyl.

A particularly preferred group of the sub-formula (e) is the phenoxyacetamido group. Another particularly preferred group of the sub-formula (e) is the phenyl-acetamido group.

Other particularly suitable groups of the formula R CO—NH— include the α - methylphenoxyacetamido, α - methyl - phenylacetamido, α - methyl - 2 - thienylacetamido, α - methyl - 3 - thienylacetamido, 2 - thienylacetamido, 3 - thienylacetamido, α - hydroxyphenylacetamido, α - hydroxy(p - hydroxyphenyl)acetamido, α - hydroxy - 2 - thienylacetamido, α - hydroxy - 3-thienylacetamido, α - aminophenylacetamido, α - amino(p - hydroxyphenyl) acetamido, α - amino - 3 - thienylacetamido, α - amino - 2 - thienylacetamido, α - carboxyphenylacetamido, α - carboxy(p - hydroxyphenyl)acetamido, α - carboxy - 2 - thienylacetamido, α - carboxy - 3 - thienylacetamido, the methyl, ethyl, propyl, butyl, phenyl, methylphenyl or indanyl ester of α - carboxyphenylacetamido, the methyl, ethyl, propyl, butyl, phenyl, methylphenyl or indanyl ester of α-carboxy (p-hydroxyphenylacetamido), the methyl, ethyl, propyl, butyl, phenyl, methylphenyl or indanyl ester of α-carboxy-2-thienylacetamido, and the methyl, ethyl, propyl, butyl, phenyl, methylphenyl or indanyl ester of α-carboxy-3-thienylacetamido.

The ureido group in either sub-formula (c) or sub-formula (f) is of the formula —$NHCONR^7R^8$ wherein $R^7$ is a hydrogen atom or a $C_{1-6}$ alkyl group and $R^8$ is $C_{1-6}$ alkyl or an optionally substituted 5- or 6-membered heteroaryl or heterocyclyl group containing one or two nitrogen atoms, or $R^7$ and $R^8$ together with the nitrogen atom to which they are joined form an optionally substituted heteroaryl or heterocyclyl ring containing 1 or 2 nitrogen atoms.

The optional substituents for $R^8$ and for the rings formed by $R^7$ and $R^8$ together include one, two or three groups selected from $C_{1-6}$ alkyl, $C_{2-6}$ alkenyl, $C_{2-6}$ alkynyl, $C_{3-6}$ cycloalkyl, phenyl, oxo, hydroxy, $C_{1-6}$ alkoxy, $C_{2-6}$ alkenyloxy, $C_{3-6}$ cycloalkyloxy phenoxy, mercapto phenylthio, $C_{1-6}$ alkylthio.amino, $C_{1-6}$ alkyl-

amino, phenylamino, benzylamino, sulphonylamino, $C_{1-6}$ alkyl sulphonylamino or p-aminosulphonylphenylamino. Alternatively two substituents on the ring $R^8$ or on the ring formed by $R^7$ and $R^8$ together may form the residue of a further carbocyclic or heterocyclic ring.

One particularly preferred sub-group of compounds is that of the formula (II):

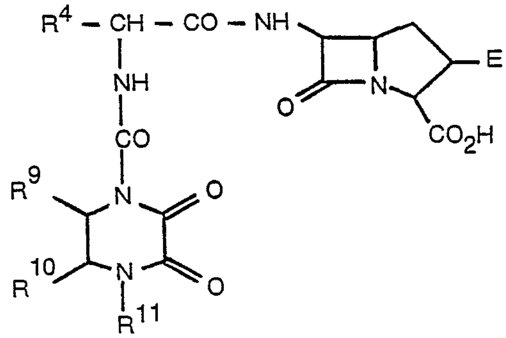

(II)

and pharmaceutically acceptable salts and cleavable esters thereof wherein E and $R^4$ are as hereinbefore defined, $R^9$ and $R^{10}$ are independently selected from hydrogen, $C_{1-6}$ alkyl, halo, amino, hydroxy or $C_{1-6}$ alkoxy, and $R^{11}$ is hydrogen or $C_{1-6}$ alkyl.

Suitably $R^{11}$ is methyl, ethyl, n-propyl, isopropyl, *sec*-butyl, isobutyl or *tert*-butyl.

Suitably $R^9$ and $R^{10}$ are independently selected from methyl, ethyl, n-propyl, isopropyl, *sec*-butyl, isobutyl or *tert*-butyl.

Preferably $R^9$ and $R^{10}$ are both hydrogen.

Preferably $R^{11}$ is ethyl.

A further preferred sub-group of compounds of this invention is that of formula (III):

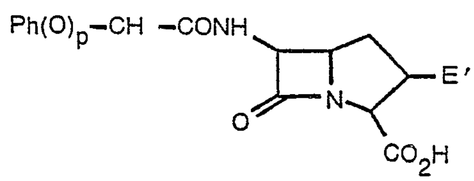

(III)

and pharmaceutically acceptable salts and cleavable esters thereof wherein E′ is a methoxycarbonyl or cyano group, and p is zero or one.

Another preferred sub-group of compounds of this invention are those of the formula (IV):

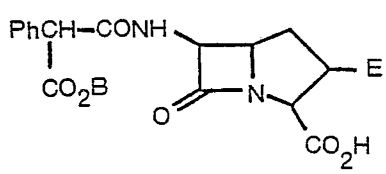

(IV)

and pharmaceutically acceptable salts and cleavable esters thereof wherein E′ is a methoxycarbonyl or cyano group, and B is a cation, hydrogen atom or a benzyl, phenyl, methyl-phenyl or indanyl group.

Yet another preferred sub-group of compounds of this invention is that of the formula (V):

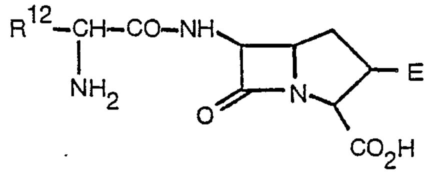

(V)

and pharmaceutically acceptable salts and cleavable esters thereof wherein E′ is a methoxycarbonyl or cyano group, and $R^{12}$ is phenyl or p-hydroxyphenyl.

A particularly preferred class of compounds of this invention is that of the formula (VI):

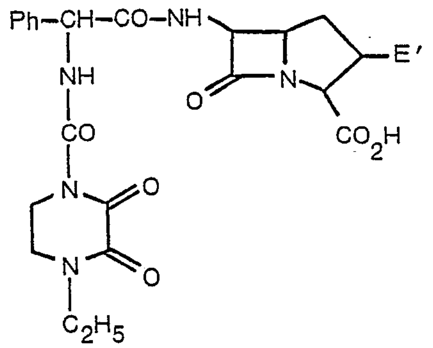

(VI)

and pharmaceutically acceptable salts and cleavable esters thereof wherein E' is a methoxycarbonyl or cyano group.

Preferred compounds of this invention include:

(2R, 5R, 6S) 1 - aza - 3 - methoxycarbonyl - 6 -[D,α - (4 - ethyl - 2,3 - dioxopiperazine - 1 - carboxylamino)phenylacetamido] - bicyclo[3.2.0]heptan - 7 - one - 2 - carboxylic acid, (2R, 5R, 6S) 1 - aza - 3 - methoxycarbonyl - 6 - phenylacetamido-bicyclo[3.2.0]heptan - 7 - one - 2 - carboxylic acid, (2R, 5R, 6S)1 - aza - 3 - methoxycarbonyl - 6 - phenoxyacetamido-bicyclo - [3.2.0)heptan - 7 - one - 2 - carboxylic acid, (2R, 5R, 6S)1 - aza - 3 methoxycarbonyl - 6 - [D,2 - (2 - (4 - aminosulphonylanilino) - 4 - hydroxypyrimidyl - 5 - aminocarbonylamino) - 2 - (4 - hydroxyphenyl)acetamido]bicyclo - [3.2.0]heptan - 7 - one- 2-carboxylic acid and (2R, 5R, 6S)1 - aza - 3 - methoxycarbonyl - 6 - [D,2 - (2 - (4 - aminosulphonylanilino) - 4 - hydroxypyrimidyl - 5 - aminocarbonylamino) - 2 - (phenyl)acetamido]-bicyclo[3.2.0.]heptan - 7 - one-2-carboxylic acid, and pharmaceutically acceptable salts thereof in particular the sodium salts.

Suitable pharmaceutically acceptable salts of the compounds of the formulae (I)—(VI) include metal salts for example aluminium, sodium, potassium calcium and magnesium, and ammonium or substituted ammonium salts.

An example of a particularly apt substituted ammonium salt is the p-toluidine salt.

Particularly apt metal salts are the sodium, potassium, calcium and magnesium salts

A preferred salt is the sodium salt.

A preferred salt is the potassium salt.

The compounds of the formulae (I)—(VI) are aptly in the form of a free carboxylic acid. If a free amino group is present in the group RCONH and the carboxylate is in the form of a free acid then a zwitterion may be formed. For example the compound of the formula (V) may be depicted as a zwitterion.

If the group E in the compound of the formula (II) contains an ester group cleavable by hydrogenolysis as hereinbelow described, then a di-acid may be formed. Such di-acids may be presented in the form of a di-salt, suitable pharmaceutically acceptable salts for a carboxy group E being as hereinbefore described.

Cleavable esters of the compounds of the present invention include those cleavable by hydrogenolysis and those cleavable by ready hydrolysis in the human body.

Suitable esters that are cleavable by hydrogenolysis include benzyl esters wherein the phenyl part is optionally substituted by a lower alkyl group of 1 to 6 carbon atoms, a lower alkoxy group of 1 to 6 carbon atoms, a chlorine or bromine atom or a nitro group.

Esters cleavable by hydrogenolysis are most useful as intermediates to other compounds of this invention.

Suitable esters that are cleavable in the human body, that is to say in-vivo hydrolysable include those of sub-formulae (g), (h) and (j):

$$-CO-O-CHR_1-O-CO-R_2 \quad (g)$$

$$-CO-O-CHR_1-O-CO-OR_2 \quad (h)$$

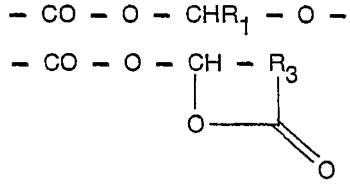

(j)

wherein $R_1$ is a hydrogen atom or a methyl group, $R_2$ is an alkyl group of up to 4 carbon atoms or a phenyl or benzyl group and $R_3$ is a —CH=CH—, 1,2-phenylene or 4,5-dimethoxy-1,2-phenylene group.

In-vivo hydrolysable esters are those esters which hydrolyse in the human body to produce the parent acid or its salt. Such esters may be identified by administration to a test animal such as a rat or a mouse by intravenous administration and thereafter examining the test animal's body fluids for the presence of the compound of the formula (I) or its salt.

Particularly apt in-vivo hydrolysable esters include the acetoxylmethyl, pivaloyloxymethyl, α-acetoxyethyl, α-pivaloyloxyethyl, α-ethoxycarbonyloxyethyl, phthalidyl and 3,4-dimethoxyphthalidyl ester.

A preferred ester group is the phthalidyl ester.

Esters of the compounds of the formula (I) if desired, may be presented in the form of their acid addition salts if an amino group is present in either of groups $R^1$ or $R^2$. The acid used to form the salt will most suitably be pharmaceutically acceptable, but non-pharmaceutically acceptable acid addition salts are also envisaged, for example as intermediates in the preparation of the pharmaceutically acceptable salts by ion exchange. Suitable pharmaceutically acceptable acid addition salts include those of inorganic and organic acids, such as hydrochloric, phosphoric, sulphuric, methanesulphonic, toluenesulphonic, citric, malic, acetic,lactic, tartaric, propionic and succinic acid.

Most suitably the acid addition salt is provided as a solid and preferably as a crystalline solid.

The compounds of the formulae (I)—(VI) are generally provided as racemic mixtures. It is believed that the optical isomer possessing greatest antibacterial activity is that depicted in the compound of the formula (Ia):

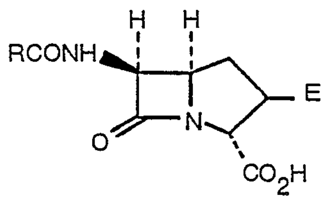

(Ia)

and pharmaceutically acceptable salts and in-vivo hydrolysable esters thereof wherein R and E are as defined in relation to a compound of the formula (I). This depicted optical isomer has the configuration 2R, 5R, 6S.

The compounds of the formulae (I)—(VI) may be provided with either the R— or S— configuration at C—3 or may be provided as a mixture thereof.

The compounds of this invention are preferably provided in substantially pure form contaminated with not more than 25% of impurities, suitably not more than 15% and preferably not more than 5%. Examples of possible impurities include other β-lactams such as those of European Patent Application Number 81300479.3 (EP - A - 34443).

In another aspect of this invention there is provided a pharmaceutical composition which comprises a compound of the formula (I) or a pharmaceutically acceptable salt of in vivo hydrolysable ester thereof as hereinbefore defined and a pharmaceutically acceptable carrier.

Such compositions are normally provided in unit dose form containing from 50 to 500 mg or more usually from 100 to 250 mg of a compound of this invention.

The compositions of this invention are usually adapted for administration to animals including humans. Such compositions may be formulated in a conventional manner for antibacterial agents, for example in a similar manner to that known for penicillins and cephalsoporins.

The compounds of this invention may be provided in orally administrable form, for example as tablets or capsules. The compounds of this invention may be provided in a form suitable for administration by injection or infusion, for example in the form of a sterile salt such as the sterile sodium salt sealed in a vial ampoule.

It is believed that infections most readily treated by the compounds of this invention are those due to strains of Bacillus, Staphylcoccus and Streptococcus.

The present invention also provides a process for the preparation of a compound of the formula (I) or pharmaceutically acceptable or cleavable ester thereof which process comprises the reduction of a compound of the formula (VII):

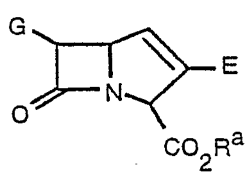

(VII)

wherein $R^a$ is a hydrogen atom or carboxy-blocking group, E is as defined in relation to formula (I) and G is a group RCONH— wherein R is as defined in relation to formula (I) or G is an azido, amino or protected amino group; and thereafter

i) if G is azido or protected amino converting G to amino,

ii) acylating any compound wherein G is amino with an N-acylating derivative of a carboxylic acid of the formula (VIII):

$$RCO_2 \qquad (VIII)$$

wherein R is as hereinabove defined and any group capable of being acylated is optionally protected:

iii) converting an ester to form an acid or pharmaceutically acceptable salt,

iv) converting an acid or salt to form a cleavable ester.

Preferably G is a group RCONH.

The reduction of the compound of the formula (VII) may be suitably effected by hydrogenation, preferably in the presence of a transition metal catalyst. The pressure of hydrogen used in the reaction may be low, medium or high but in general an approximately atmospheric or slightly super-atmospheric pressure of hydrogen is preferred. The transition metal catalyst employed is preferably palladium on charcoal or on calcium carbonate. The hydrogenation may be effected in any inert solvent in which the ester is soluble such as aqueous dioxan or the like. If this hydrogenation is performed on a compound of the formula (VII) wherein $R^a$ is a salt-forming cation or hydrogenolysable group and is carried out in the presence of a base then a salt of the compound of the formula (I) is produced. Suitable bases for inclusion include $NaHCO_3$, $KHCO_3$, $Na_2CO_3$, $K_2CO_3$, $CaCO_3$, $MgCO_3$, $LiHCO_3$, $NH_4OCOCH_3$ and the like. If no base is present then hydrogenation leads to the preparation of an acid within formula (I) which may then be neutralised if desired to yield a salt. Suitable bases which may be used to neutralise acids within formula (I) include LiOH, NaOH, $NaHCO_3$, KOH, $Ca(OH)_2$ and $Ba(OH)_2$.

An alternative method of hydrogenation suitable for use in this procedure is catalytic transfer hydrogenation using a transition metal catalyst. The transition metal catalyst employed is preferably palladium, for example palladium on carbon, palladium on barium sulphate or palladium black. It is preferred to use palladium on carbon, for example 10% palladium on charcoal. Suitable solvents include those in which the compound is soluble, for example ethanol, dimethylformamide, dimethylacetamide and mixtures thereof. In catalytic transfer hydrogenation a hydrogen donor is used (not hydrogen gas). Suitable hydrogen donors include cyclohexene and 1, 4-cyclohexadiene. The reaction is conveniently performed at an elevated temperature suitably between ambient and 100°C, more suitably between 30°C and 80°C. It is sometimes suitable to perform the reaction at reflux temperature.

The term "acylating derivative of a carboxylic acid" includes any N-acylating compound suitable for the performance of analogous reactions with 6-aminopenicillanic acid or 7-aminocephalosporanic acid or salts or esters thereof, for example an acid halide, a mixed anhydride or other reactive derivative such as that produced by the reaction of an acid with an enzyme or a condensation-promoting agent such as dicyclohexylcarbodi-imide or its chemical equivalent. Reactive groups present in such acylating agents may be protected in conventional manner.

Suitable N-acylating compounds may be summarised by the formula (IX):

$$RCO—W \qquad (IX)$$

wherein R is as defined in relation to a compound of the formula (I) and W is a readily displaceable group. Most suitably W is a bromine atom. Preferably W is a chlorine atom.

Reduction of the azido group to an amino group may be performed in conventional manner.

Suitably G is a protected amino group, which is protected in conventional manner known in the art.

Any group R that contains a reactive moiety such as $NH_2$ or $CO_2H$ may be suitably protected during the process of this invention. For example an amino group may be reacted with a p-nitrobenzyloxycarbonyl halide to provide a p-nitrobenzyloxycarbonylamino moiety which is cleavable on hydrogenation to yield an amino moiety.

Suitable carboxyl-blocking derivatives for the group $—CO_2R^a$ in formulae (VII) include salts, esters, and anhydride derivatives of the carboxylic acid. The derivative is one which may readily be cleaved at a later stage of the reaction. The salts need not be pharmaceutically acceptable. Suitable salts include inorganic salts, for example metal salts such as silver or mercuric salt, or alkali metal salts such as the lithium or sodium salt, and tertiary amine salts.

Suitable ester-forming carboxyl-blocking groups are those which may be removed under conventional conditions, and include those of sub-formulae (L)—(M) as hereinbefore defined. Such groups for $R^a$ include benzyl, p-methoxybenzyl, 2,4,6 - trimethylbenzyl, 3,5 - di - t - butyl - 4 - hydroxybenzyl, benzoylmethyl, p-nitrogenzyl, 4 - pyridyl - methyl, 2,2,2 - trichloroethyl, 2,2,2 - tribromoethyl, acetonyl, 2-methylallyl, t-butyl, 5-amyl, diphenylmethyl, triphenylmethyl, methyl, ethyl, allyl, adamantyl, o-nitrobenzyl, 2-benzyloxphenyl, 4-methylthiophenyl, tetrahydrofur-2-yl, tetrahydropyran - 2 - yl, methoxymethyl, a silyl, stannyl or phosphorus-containing group, or an *in-vivo* hydrolysable ester.

The carboxyl group may be regenerated from any of the above esters by usual methods appropriate to the particular $R^a$ group, for example, acid — and base — catalysed hydrolysis, or by enzymically-catalysed hydrolysis, photolysis or by hydrogenation. The hydrolysis must of course by carried out under conditions to which the groups on the rest of the molecule are stable.

The salts of acids of formula (I) may be converted to esters in conventional manner. Suitable methods include the reaction of an alkali metal salt such as a sodium or potassium salt with a reactive halide or sulphonate ester such as a bromide, chloride, mesylate or tosylate, fór example bromophthalide. Such esterification may be carried out under conventional conditions, for example in dimethylformamide at room temperature.

The compounds of the formula (VII) may be prepared by the methods disclosed in European Patent

Application 81300479.3 (Publication Number 0034443).

The following examples illustrate the invention. For compounds *, one enantiomer is depicted for convenience.

## Example 1

Benzyl 3,3-Dimethyl-2-nitroacrylate (1)

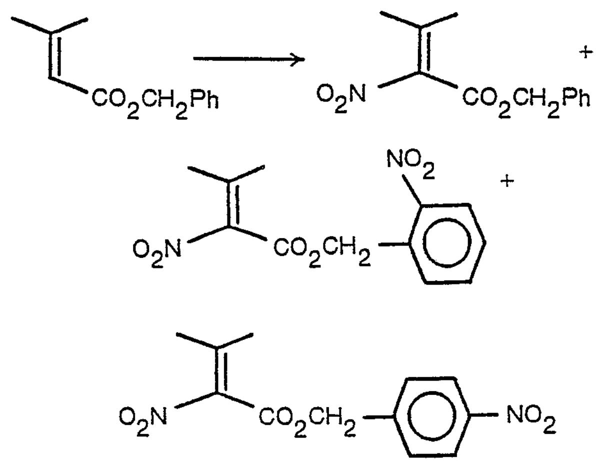

(1)

(2)

(3)

Benzyl 3,3-dimethylacrylate (80 g) was added dropwise to a well stirred mixture of fuming nitric acid (157 ml. 95%) and water (34 ml) at −20° over 1 h, (cf. A.G. Brown and T.C. Smale, *J. Chem. Soc. Perkin 1,* 65, 1972). After 2 h at −20° and warming to 0° for 2 h, the reaction mixture was poured into ice (800 ml) and extracted with chloroform (3×200 ml). The combined organic extracts were washed successively with water (3×200 ml), saturated sodium hydrogencarbonate solution (2×350 ml), brine (2×200 ml), dried ($MgSO_4$) and evaporated to an oily residue. Chromatography on silica H gave the product (1; 36 g) as an oil $\nu_{max.}$ (film) 1715, 1650, 1530, 1370 $cm^{-1}$; ppm ($CDCl_3$) 1.98 (3H, s), 2.23 (3H, s), 5.20 (2H, s), 7.40 (5H, m). (Found: C, 61.2; H, 5.7; N, 6.0; $C_{12}E_{13}NO_4$ requires C, 61.3; H, 5.5; N, 6.0%).

Further elution of the column provided the *o*-nitro-benzyl 2-nitro-3,3-dimethyl acrylate (2), and *p*-nitrobenzyl 2-nitro-3,3-dimethyl acrylate (3) as white solids (47 g). *Ortho* isomer (21) $\nu_{max.}$ ($CHCl_3$) 1735, 1650, 1530, 1350 $cm^{-1}$; δ ppm ($CDCl_3$) 2.03 (3H, s), 2.30 (3H, s), 5.73 (2H, s), 7.4—7.8 (4H, m), *para* isomer (3) $\nu_{max.}$ ($CHCl_3$) 1733, 1650, 1530, 1350 $cm^{-1}$; δ ppm ($CDCl_3$) 2.03 (3H, s), 2.30 (3H, s), 5.36 (2H, s), 7.52 and 8.30 (4H, ABq, J 9Hz).

## Example 2

Benzyl 2-Amino-(N-cinnamylidene)-3,3-dimethyl acrylate (5)

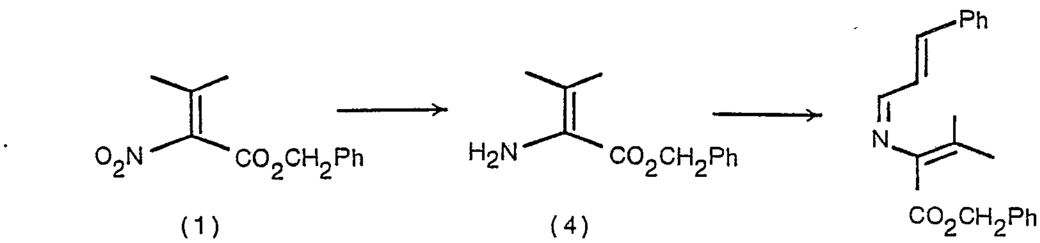

(1) (4) (5)

Benzyl 3,3-dimethyl-2-nitroacrylate (1; 17.2 g) was converted to benzyl 2-amino-3,3-dimethylacrylate (4; 12.2 g) using the same conditions as for the corresponding methyl ester (A. G. Brown and T. C. Smale, *J. Chem. Soc. Perkin I,* 65, 1972). $\nu_{max.}$ ($CHCl_3$) 1710, 1690, 1640 $cm^{-1}$; δ ppm ($CDCl_3$) 1.70 (3H, s), 2.05 (3H, s), 2.8—3.7 (2H, m, exch.), 5.17 (2H, s), 7.31 (5H, s). The crude product (4; 80% pure by tlc and nmr) in dry methylene dichloride (100 ml) was vigorously stirred with *trans*-cinnamaldehyde (7.72 g) and anhydrous magnesium sulphate (7g) for 17h. The mixture was filtered, evaporated, and dried *in vacuo* to give the Schiff base (5) as a red gum (19.7 g); $\lambda_{max.}$ (EtOH) 297mm (ε27600); $\nu_{max.}$ ($CHCl_3$) 1710, 1675, 1625 $cm^{-1}$; δ ($CDCl_3$) 1.91 (3H, s), 2.00 (3H, s), 5.26 (2H, s), 6.5—7.6 (7H, m), and 7.75 (1H, d, *J* (7Hz).

Example 3

*cis* 3-Azido-1-(1-benzyloxycarbonyl-2-methylprop-1-enyl)-4-styryl-azetidin-2-one (6)

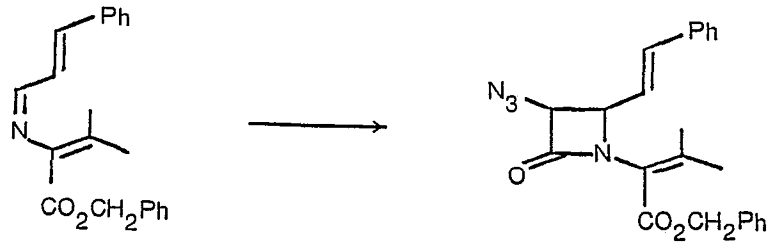

(5) (6)

Azidoacetic acid (9.36 g) was dissolved in dry methylene dichloride (80 ml) at 0° under argon and trifluoroacetic anhydride (13.05 ml) added dropwise over 10 min. After 15 min. triethylamine (12.99 ml) in methylene dichloride (20 ml) was carefully added dropwise, over 15 min., and stirring at 0° continued a further 45 min. The solution was transferred under argon to a dropping funnel, cooled to −76°, and added over 1h to a mixture of the Schiff base (5) (19.7 g) and triethylamine (12.99 ml) in methylene dichloride (160 ml) at 0°. After a further 1h at 0° the solution was diluted with methylene dichloride, washed successively with water, dilute aqueous sodium hydrogencarbonate, brine, dried ($MgSO_4$) and evaporated. Chromatography of the residue on silica H afforded the product (6; 12.6 g) as a red-orange gum; $\lambda_{max.}$ (EtOH) 256 mm (ε23,400) $\nu_{max.}$ ($CHCl_3$) 2115, 1760, 1720cm$^{-1}$; δ ppm ($CDCl_3$) 1.98 (3H, s), 2.21 (3H, s), 4.63 (1H, dd, *J* 7 and 10Hz), 4.79 (1H, d, *J* 7Hz), 5.08 and 5.32 (2H, ABq, *J* 15Hz), 6.11 (1H, dd, *J* 10 and 20Hz), 6.48 (1H, d, *J* 20Hz), 7.29 (5H, s), 7.37 (5H, s). (Found: C, 68.4; H, 5.5; N, 14.0; $C_{23}H_{22}N_4O_3$ requires C, 68.7; H, 5.5; N, 13.9%).

Example 4

*cis* 1-(1-Benzyloxycarbonyl-2-methylprop-1-enyl)-3-phenoxyacetamido-4-styryl-azetidin-2-one (8)

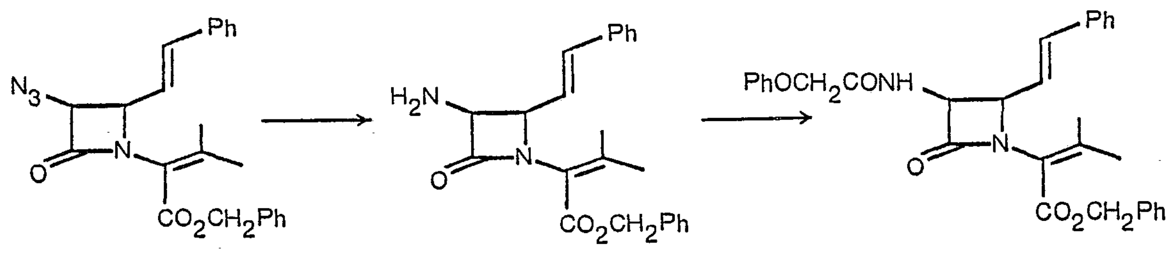

(6) (7) (8)

To the β-lactam (6; 6.38 g) in dry methylene dichloride (125 ml) at 0° was added triethylamine (2.45 ml). Hydrogen sulphide was bubbled through the mixture for 5 min. and the resulting dark solution stood at 0° for 1.5h. The solvent was then removed under reduced pressure and the residue re-evaporated (× 3) from methylene dichloride, to afford the crude amine (7) an an orange solid. Without further purification, the β-lactam (7) was dissolved in dry methylene dichloride (60 ml) at −20° and triethylamine (2.45 ml) added, followed by dropwise addition of phenoxyacetyl chloride (2.45 ml) in methylene dichloride (5 ml) over 10 min. The solvent was evaporated and the residue taken up in ethyl acetate, washed successively with water, dilute aqueous sodium hydrogencarbonate, brine, dried ($MgSO_4$) and evaporated to a gum. Chromatography on silica H provided the product (8; 5.5 g) as a white crystalline solid, m.p./ 107—108° (ethyl acetate — light petroleum 60—80°), $\lambda_{max.}$ (EtOH) 258 nm (23,200); $\nu_{max.}$ ($CHCl_3$ 3245, 1755, 1690, 1650 sh, 1630 sh, cm$^{-1}$; δ ppm ($CDCl_3$) 2.05 (3H, s), 2.18 (3H, s), 4.44 (2H, s), 4.67 (1H, dd, *J* 5 and 8Hz), 5.08 and 5.27 (2H, ABq, *J* 12Hz), 5.20 (1H, dd, *J* 5 and 8Hz), 6.04 (1H, dd, *J* 8 and 16Hz), 6.47 (1H, d, *J* 16Hz), 6.7—7.5 (10H, m), 7.63 (1H, d, *J* 8Hz, exch.). (Found: C, 72.7; H, 5.9; N, 5.4; $C_{31}H_{30}N_2O_5$ requires C, 72.9; H, 5.9; N, 5.5%).

Example 5

*cis* 1-(1-Benzyloxycarbonyl-2-methylprop-1-enyl)-4-(1,1-dimethoxymethyl)-3-phenoxyacetamido-azetidin-2-one (10)

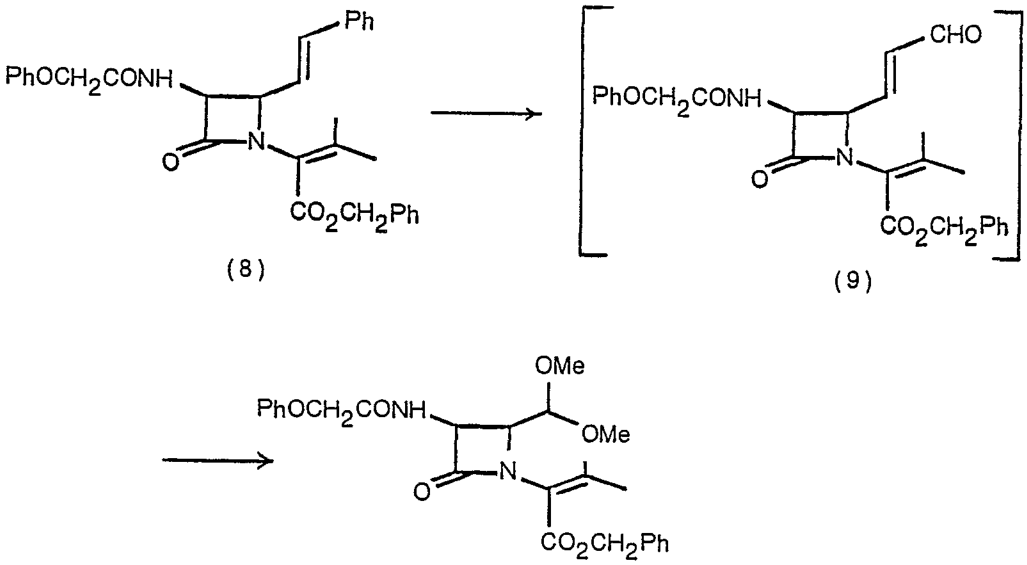

(8) (9) (10)

Ozonised oxygen was bubbled through a solution of the β-lactam (8; 3.06 g) in methylene dichloride (30 ml) at −76° for 1.5 h when cleavage of the 4-styryl moiety was complete. Argon was then bubbled through the solution for 20 min followed by the addition of triphenylphosphine (1.56 g) in a little methylene dichloride, and the reaction allowed to reach ambient temperature. After 1.5 h the solvent was removed and the residual aldehyde (9) immediately taken up in methanol (50 ml) and 2,2-dimethoxypropane (20 ml). The solution was refluxed for 16 h in the presence of p-toluene sulphonic acid as catalyst. The reaction mixture was diluted with ethyl acetate, washed with dilute aqueous sodium hydrogencarbonate, brine, dried ($MgSO_4$) and evaporated to a residual oil. Chromatography on silica H provided the product (10; 2.6 g) $\nu_{max.}$ ($CHCl_3$) 3420, 1755, 1715 sh, 1685, 1630 $cm^{-1}$; δ ppm ($CDCl_3$) 2.02 (3H, s), 2.20 (3H, s), 3.22 (6H, s), 4.17 (1H, dd, *J* 3 and 6 Hz), 4.33 (1H, d, *J* 3Hz), 4.60 (2H, s), 5.08 and 5.30 (2H, ABq, *J* 12 Hz)., 5.47 (1H, dd, *J*6 and 10Hz), 6.8—7.5 (10H, m), 7.6 (1H, d, *J* 10 Hz). (Found: C, 64.7; H, 6.2; N, 5.8; $C_{26}H_{30}N_2O_7$ requires C, 64.7; H, 6.2; N, 5.8%).

Example 6

*cis* 4-(1,1-Dimethoxymethyl)-1-(1-hydroxy-1-benzyloxycarbonyl-methyl)-3-phenoxyacetamido-azetidin-2-one (11)

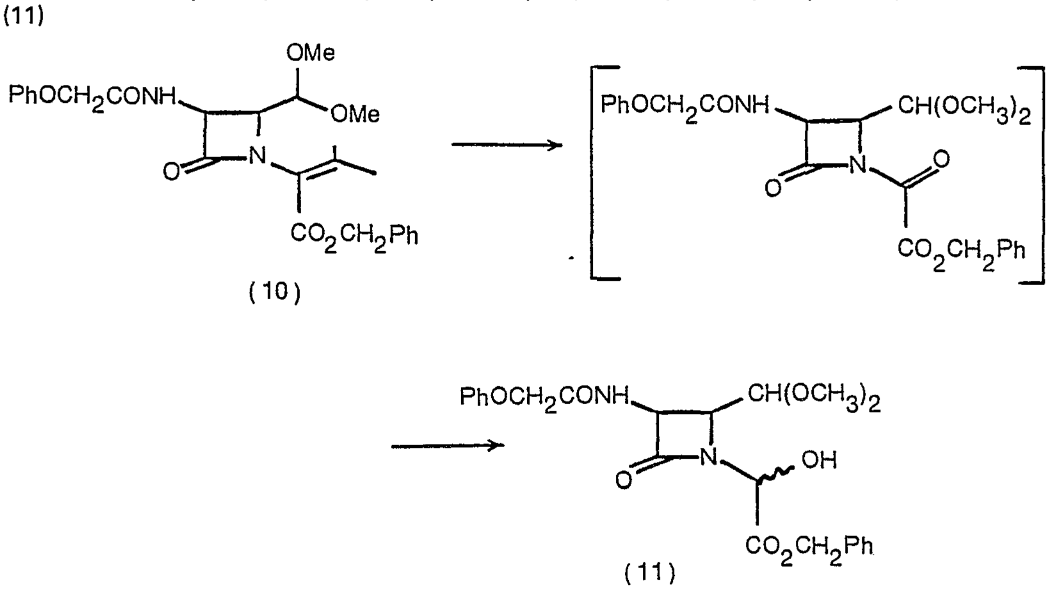

(10) (11)

Ozonised oxygen was bubbled through the β-lactam (10; 2.4 g) in dry methylene dichloride (20 ml) at −20° for 30 min. Argon was then bubbled through the solution for 10 min. which was then equilibrated with ambient temperature over 45 min. Glacial acetic acid (15 ml) was added, followed by activated zinc dust (3.6

g) portionwise with occasional cooling over 10 min. After 2h at zoom temperature the mixture was filtered, diluted with ethyl acetate, and washed successively with water (×3), saturated aqueous sodium hydrogencarbonate (×2), brine, dried ($MgSO_4$) and evaporated. Chromatography on silica H afforded the less polar isomer (11) (900 mg), $\nu_{max.}$ ($CHCl_3$) 3500, 3420, 1780, 1758 and 1690 $cm^{-1}$; δ (250 MHz) ($CDCl_3$) 3.31 (3H, s), 3.32 (3H, s), 3.85 (1H, dd, *J*, 2.7 and 5.5Hz), 4.24 (1H, d, *J* 2.7Hz), 4.26 (1H, d, *J* 10.5Hz, exch. $D_2O$), 4.47 and 4.55 (2H, ABq, *J* 14.9Hz), 5.19 and 5.29 (2H, ABq, *J* 12.1Hz), 5,52 (1H, dd *J* 5.5 and 10.5Hz), 5.63 (1H, d, *J* 10.5Hz, becomes s on $D_2O$ exch.), 6.85—7.4 (10H, series of m) and 7.51 (1H, d, *J* 10.5Hz). (Found: C, 60.3; H, 6.0; N, 6.1. $C_{23}H_{26}N_2O_8$ requires C, 60.3; H, 5.7; N, 6.1%).

Further elution provided the more polar isomer (11) (1 g), $\nu_{max.}$ ($CHCl_3$) 3500, 3415, 1780, 1750sh and 1690 $cm^{-1}$; δ (250MHz) ($CDCl_3$) 3.22 (3H, s), 3.35 (3H, s), 4.05 (1H, d, *J* 6.6Hz, exch. $D_2O$), 4.15 (1H, dd, *J* 3.9 and 5.5Hz), 4.3 (1H, d, *J* 3.9Hz), 4.48 and 4.54 (2H, ABq, *J* 15.2Hz), 5.2 and 5.31 (2H, ABq, *J* 12.1Hz), 5.43 (1H, d, *J* 6.6Hz, collapses to s on exch. $D_2O$), 5.51 (1H, dd, *J* 5.5 and 9.7Hz), 6.9—7.4 (10H, series of m), and 7.5 (1H, d, *J* 9.7Hz). (found: C, 60.2, H, 5.7; N, 6.2%).

## Example 7

*cis* 1-[1-Benzyloxycarbonyl-2-methoxycarbonyl-2-triphenylphosphoranylidene-ethyl]-4-(1,1-dimethoxymethyl)-3-phenoxyacetamido-azetidin-2-one (13)

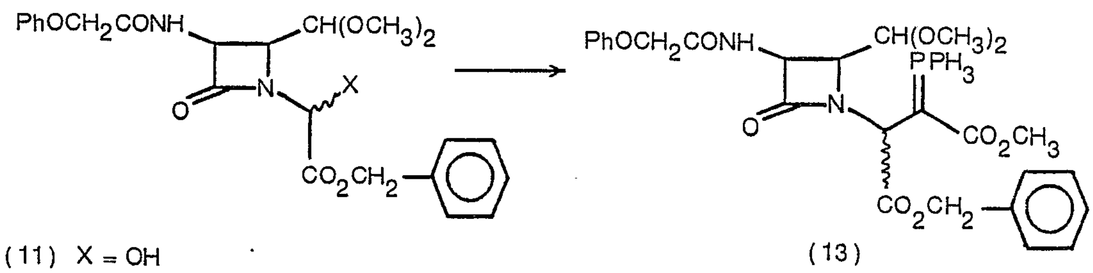

(11) X = OH

(13)

(12) X = Cl

The hydroxy-compound (11) (210 mg) was dissolved in dry tetrahydrofuran (10 ml) and cooled to −20°C. 2,6-Lutidine (74 mg) was added, followed by dropwise addition of thionyl chloride (82 mg) in dry tetrahydrofuran (1 ml) during 10 min. After 10 min. the mixture was diluted with dry toluene, filtered, and the filtrate was evaporated and dried *in vacuo* for 15 min. to give (12) as a gum.

The total crude product (12) was dissolved in dry methylene dichloride (10 ml) containing carbomethoxymethylenetriphenylphosphorane (337 mg). The solution was left at room temperature under argon for 40h, then evaporated and the residue chromatographed on silica H to give the product (13) as an inseparable mixture of isomers (153 mg), $\nu_{max.}$ ($CHCl_3$) 3390, 1755, 1678, 1620$cm^{-1}$.

## Example 8

(2RS, 5RS, 6RS) Benzyl 1-Aza-3-methoxycarbonyl-6-phenoxyacetamido-bicyclo-[3.2.0]-hept-3-en-7-one-2-carboxylate (14) and *cis* 1-[(1RS-1-Benzyloxycarbonyl-2-methoxycarbonyl-2-triphenylphosphoranylidene-ethyl]-4-formyl-3-phenoxyacetamido-azetidin-2-one (15)

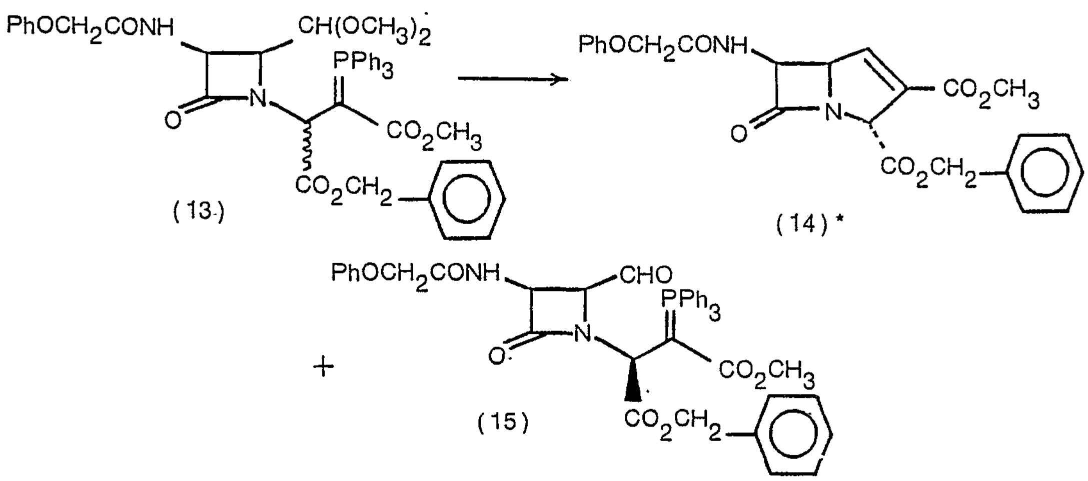

(13) (14)*

\+

(15)

The azetidinone (13) (54 mg) was dissolved in 95% aqueous trifluoroacetic acid (1 ml) at room temperature, and after 3h. the solution was evaporated, toluene added and the process repeated (x2). The residue

was taken up in ethyl acetate and the solution washed successively with aqueous $NaHCO_3$, brine, dried ($MgSO_4$) and evaporated. Chromatography on silica H provided the product (14) (13 mg), $\lambda_{max.}$ (ETOH) 261 (ε2332), 268 (2219), 275nm (1623); $\nu_{max.}$ ($CHCl_3$) 3410, 1790, 1735 and 1690cm$^{-1}$; δ ppm ($CDCl_3$) 3.65 (3H, s), 4.50 (2H, s), 4.98 (1H, dd, *J* 5.5, 3.3 and ~1.5Hz), 5.16 (2H, s), 5.37 (1H, dd, *J* 3.3 and ~1.8Hz), 5.54 (1H, dd, *J* 7 and 5.5Hz), 6.80 (1H, dd, *J* 1.8 and 1.5Hz) and 6.8—7.4 (11H, m). (Found: C, 63.7; H, 5.2; N, 5.8. $C_{24}H_{22}N_2O_7$ requires C, 64.0; H, 4.9; N, 6.2%). (Found: $M^+$—$CH_2Ph^{7+}$, 359.0879, $C_{17}H_{15}N_2O_7$ requires M—$CH_2Ph^{-4}$, 359.0873).

Further elution of the column provided the aldehyde (15) (35 mg) (contaminated with some triphenylphosphine oxide), $\nu_{max.}$ ($CHCl_3$) 3375hr, 1760, 1725, 1685 and 1615 cm$^{-1}$.

## Example 9

(2SR, 5RS, 6SR) Benzyl 1-Aza-3-methoxycarbonyl-6-phenoxyacetamidobicyclo-[3.2.0]-hept-3-en-7-one-2-carboxylate (16)

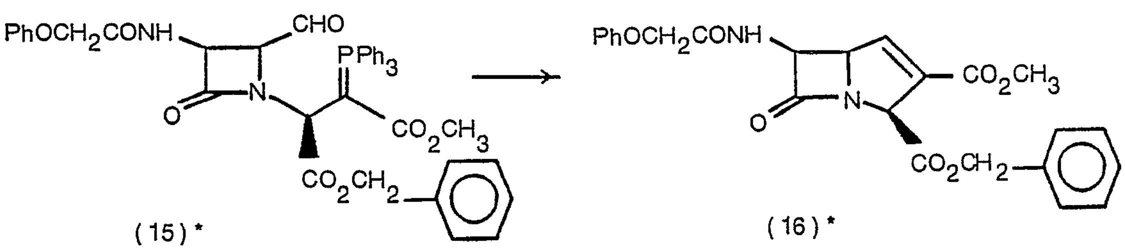

(15)*　　(16)*

The aldehyde (15) (35 mg) (contaminated with triphenylphosphine oxide) was gently refluxed in ethyl acetate for 5h, the solution cooled and evaporated to give an oil. Chromatography on silica H provided the product (16) as an amorphous white solid (15 mg) $\lambda_{max.}$ (EtOH) 261 (ε2476), 268 (2517), 275nm (1982); $\nu_{max.}$ ($CHCl_3$) 3410, 1785, 1735 and 1690 cm$^{-1}$; δ ppm ($CDCl_3$) 3.64 (3H, s), 4.50 (2H, s), 4.83 (2H, m), 5.22 (2H, s), 5.43 (1H, m), 6.79 (1H, m), 7.03 (1H, d, *J* 7Hz) and 6.8—7.4 (10H, m). (Found: C, 64.0; H, 5.2; N, 5.7; *M*$^+$ 450.1425. $C_{24}H_{22}N_2O_7$ requires C, 64.0; H, 4.9; N, 6.2%, *M* 450.1433).

## Example 10

(2RS, 5RS, 6SR) 1-Aza-3-methoxycarbonyl-6-phenoxyacetamidobicyclo [3.2.0] -heptan-7-one-2-carboxylic acid (17) and (2RS, 5RS, 6SR) methyl 1-Aza-3-methoxycarbonyl-6-phenoxyacetamidobicyclo [3.2.0]-heptan-7-one-2-carboxylate (18)

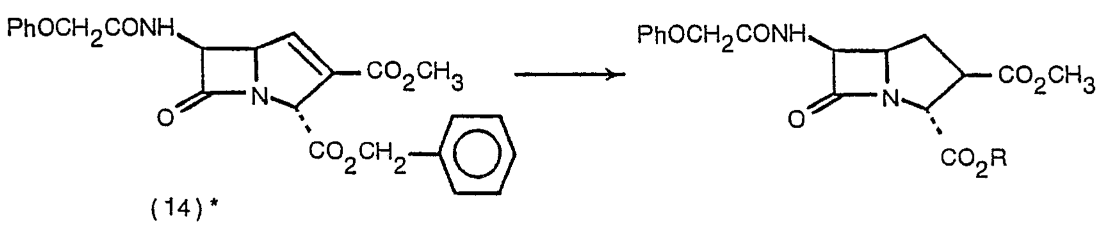

(14)*

(17)*　R = H

(18)*　R = $CH_3$

The ester (14) (100 mg) was dissolved in dioxan (12 ml) and water (3 ml) and hydrogenated using 10% Pd/C catalyst (50 mg) for 2H. The reaction mixture was filtered, and the filtrate evaporated to dryness, followed by re-evaporation from ethanol, then toluene. Trituration with ether gave a white solid (17) (80 mg), $\lambda_{max.}$ (EtOH) 262 (ε1644), 268 (1675), 275nm (1279); $\nu_{max.}$ (KBr) 3400br, 1770, 1725 and 1670 cm$^{-1}$; δ ppm [$(CD_3)_2SO$] *inter alia* 1.88—2.2 (2H, m), 3.62, 3.64 (s, together 3H), 4.28 (1H, d, *J* 7Hz), 4.59 (2H, s), 5.58 (1H, dd, *J* 5 and 9Hz), 6.9—7.1 (3H, m), 7.2—7.4 (2H, m) and 8.87 (1H, d, *J* 9Hz).

The minimum inhibitory concentration (MIC) of this compound required to inhibit the growth of various bacteria in nutrient broth are tabulated below:—

| Organism | MIC (µg/ml) |
|---|---|
| E. coli | 250 |
| Klebsiella aerogenes | 250 |
| Proteus mirabilis | 62 |
| S. typhimurium | 250 |
| B. subtilis | 2 |
| Staph. aureus Oxford | <0.5 |
| Staph. aureus Russell* | 62 |
| Strep. faecalis | 8 |
| Strep. pyogenes | <10.5 |

*β-lactamase-producing benylpenicillin-resistant strain.

The acid (17) (60 mg) was dissolved in ethyl acetate (10 ml) and dimethyl suphoxide (2 ml) and an ethereal solution of diazomethane added. Argon was then bubbled through the solution and the reaction mixture washed with water (× 3), brine, dried ($MgSO_4$) and evaporated. Chromatography on silica H gave the product (18) (31 mg), $\lambda_{max.}$ (EtOH) 262 (ε1070), 268 (1388), 275nm (1195); $\nu_{max.}$ ($CHCl_3$), 3410, 3350, 1785, 1745 and 1690 $cm^{-1}$; δ ppm (250MHz) ($CDCl_3$) 2.16 (2H, ABq, *J* 14Hz, higher field arm further coupled, t, *J* 5Hz; lower field arm further coupled, t, *J* 8Hz), 3.54 (1H, ddd, *J* 8, 5 and 3.5Hz), 3.69, 3.72, 3.77, 3.79 (s, together 6H), 4.29 (1H, ddd, *J* 8, 6 and 5Hz), 4.55, 4.57 (s, together 2H), 4.82 (1H, d, *J* 3.5Hz), 5.68 (1H, dd, *J* 9 and 5Hz), 6.8—7.4 (5H, series of multiplets) and 8.08 (1H, D, *J* 9Hz). (Found: $M^+$ 376.1282, $C507_8H_{20}N_2O_7$ requires *M* 376.1270).

Example 11

N-Cinnamylidene-p-methoxymethoxyaniline (20)

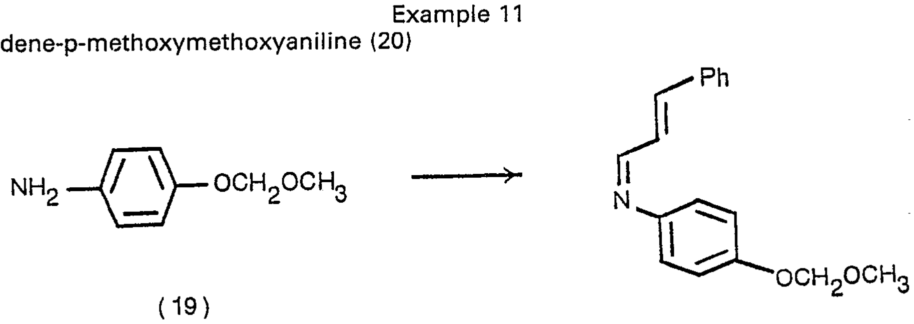

(19)

(20)

*p*-Methoxymethoxyaniline (19: T. Fukuyama, R. K. Frank and C. F. Jewell, Jr., *J. Amer. Chem. Soc.*, 1980, *102,* 2122) (13.8 g) was dissolved in dry methylene chloride (200 ml) under argon, and cinnamaldehyde (13.3 g) and dry magnesium sulphate (10 g) added. The mixture was stirred at room temperature for 15 h, filtered, and the filtrate evaporated to give a yellow solid (20) (24 g) m.p. 88—88.5° (ethyl acetate/light petroleum) $\lambda_{max.}$ (EtOH) 296 (ε23,245) and 345 n.m. (23, 206); $\nu_{max.}$ ($CHCl_3$) 1625 and 1605 $cm^{-1}$; δ ppm ($CDCl_3$) 3.47 (3H, s), 5.16 (2H, s), 6.95—7.6 (9H, m), 8.28 (1H, dd, *J* 2 and 4 Hz). (Found: C, 76.1; H, 6.5; N, 5.0; $C_{17}H_{17}NO_2$ requires C, 76.4; H, 6.4; N, 5.2%).

Example 12

cis 3-Azido-1-(p-methoxymethoxyphenyl)-4-styryl-azetidin-2-one (2)

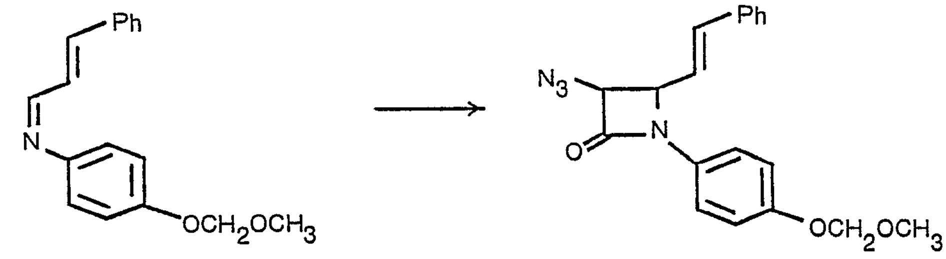

(20) (21)

The Schiff base (20) (24 g) was converted into (21) (26.1 g) as described in Example 2, m.p. 111—112°C; (ethyl acetate/light petroleum) $\nu_{max.}$ ($CHCl_3$) 2120, 1755 and 1650 $cm^{-1}$; δ ppm ($CDCl_3$) 3.38 (3H, s), 4.7—4.95 (2H, m), 5.06 (2H, s), 6.19 (1H, dd, *J* 7 and 17 Hz), 6.81 (1H, d, *J* 17 Hz) and 6.9—7.5 (9H, m). (Found: C, 65.1; H, 5.1; N, 15.9; $C_{19}H_{18}N_4O_3$ requires C, 65.1; H, 5.1; N, 16.0%).

Example 13

cis 3-Azido-4-styryl-azetidin-2-one (22)

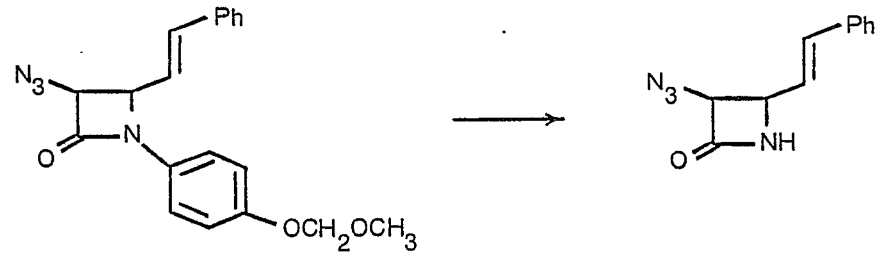

(22)

(21)

The lactam (21) (350 mg) in tetrahydrofuran (10 ml) was cooled to 0°C and ceric ammonium nitrate (274 g) in water (4 ml) added dropwise over 2—3 min to the vigorously stirred solution (see ref. in Example 11). After 10 min. solid sodium sulphite was added to decolourise the mixture, which was then poured into ethyl acetate and dilute sodium hydrogencarbonate. The organic layer was separated, washed with brine, dried and evaporated. Chromatography of the residue on silica afforded the product (22) (149 mg), m.p. 106° (ethyl acetate/light petroleum) $\nu_{max.}$ ($CHCl_3$) 3400, 2110, 1775 and 1650 $cm^{-1}$; δ ppm ($CDCl_3$) 4.48 (1H, dd, *J* 4.4 and 7 Hz), 4.81 (1H, dd, *J* 1.8 and 4.4 Hz, becomes d, *J* 4.4 Hz on irradiation at δ 6.56), 6.19 (1H, dd, *J* 7 and 16 Hz), 6.566 (1H, s), 6.69 (1H, d, *J* 16 Hz) and 7.2—7.5 (5H, m). (Found: C, 61.7; H, 4.7; N, 25.9; $C_{11}H_{10}N_4O$ requires C, 61.7; H, 4.7; N, 26.2%).

Example 14

cis 3-Phenoxyacetamido-4-styryl-azetidin-2-one (23)

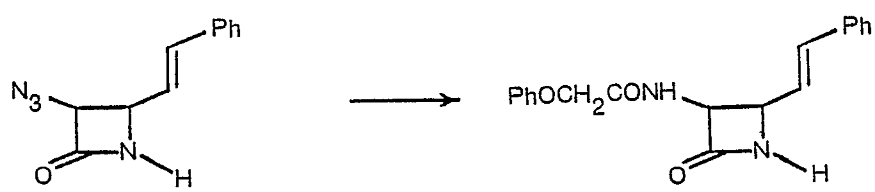

(22) (23)

The azide (22) (75 mg) was dissolved in dry methylene chloride (10 ml) at 0°C, and triethylamine (80 mg) added. Hydrogen sulphide was bubbled through the solution for 5 min. and the solution allowed to warm to room temperature. After 2 h the solvent was evaporated, the residue dissolved in methylene chloride and the process repeated.

The crude amine was dissolved in methylene chloride at −10°C and triethylamine (44 mg) added, followed by phenoxyacetyl chloride (65 mg) in methylene chloride (1 ml). The solution was washed with

dilute citric acid, brine, dried and evaporated. Chromatography on silica eluting with methylene chloride/ethyl acetate gave the product (23) (70 mg); m.p. 181—182° (ethyl acetate); $\lambda_{max.}$ (EtOH) 258 n.m. (ε 19,200); $\nu_{max.}$ (Nujol) 3290, 3225, 1780, 1730 and 1680 $cm^{-1}$; δ ppm ($[CD_3]_2SO$) (250M Hz) 4.43 (1H, dd, *J* 8.2 and 5Hz), 4.49 and 4.57 (2H, $^{B}$Bq, *J* 15Hz), 5.27 (1H, ddd, *J* 9.2, 5 and *ca.* 0.5Hz), 6.38 (1H, dd, *J* 16 and 8.2Hz), 6.65 (1H, d, *J* 16Hz), 6.8—7.5 (10H, m), 8.53 (1H, d, *J* *ca.* 0.5Hz, exch. $D_2O$) and 8.88 (1H, d, *J* 9.2Hz, exch. $D_2O$). (Found: C, 70.8; H, 5.7; N, 8.5. $C_{19}H_{18}N_2O_3$ requires C, 70.8; H, 5.6; N, 8.7%).

Example 15

*cis*-1-(1-Benzyloxycarbonyl-1-hydroxymethyl)-3-phenoxyacetamido 4-styryl-azetidin-2-one (23)

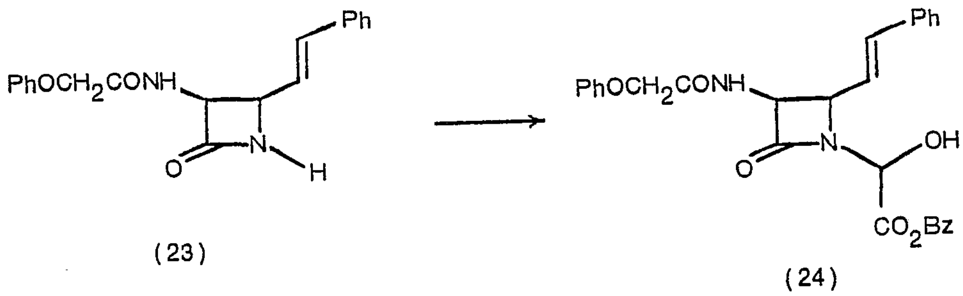

(23)

(24)

Benzyl glyoxylate (1.23 g) was dissolved in dry benzene (120 ml) and the solution refluxed for 1h with provision for the removal of water. The lactam (23) (1.98 g) was then added in dioxan (20 ml) and the reflux continued for a furher 1h. The solution was cooled to room temperature and triethylamine (62 mg) added. After 2h the solvents were evaporated off and the residue dissolved in toluene (50 ml) and allowed to crystallise at 0°C. Filtration afforded the product (24) as a mixture of isomers (2.23 g). The mother liquors from the crystallisation were evporated and chromatographed on silica to give the less polar isomer (24) (385 mg) m.p. 149—152° (ethyl acetate-hexane), $\lambda_{max.}$ ($CHCl_3$) 3500, 3415, 1770, 1750, 1690 and 1655sh $cm^{-1}$; δ ppm 4.25br (s, exch. $D_2O$), 4.38 (2H, s), 4.47 (1H, dd, *J* 5.5 and 8Hz), 5.27 (2H, AA system), 5.4 (1H, dd, *J* 5.5 and 9Hz), 5.42 (1H, s), 6.03 (1H, dd, *J* 8 and 16Hz), 5.53 (1H, d, *J* 16Hz), and 6.7—7.5 (11H, m). (Found: C, 68.9; H, 5.5; N, 5.6. $C_{28}H_{26}N_2O_6$ requires C, 69.1; H, 5.3; N, 5.8%).

Further elution provided the more polar isomer (24) (250 mg), m.p. 150—156° (ethyl acetate-hexane); $\lambda_{max.}$ ($CHCl_3$) 3500, 3410, 1770, 1750, 1690 and 1655 $cm^{-1}$; δ ppm ($CDCl_3$) 4.4 (2H, s), latter signal obscures 1H broad singlet, 4.7 (1H, dd, *J* 5.5 and 8Hz), 4.92 and 5.14 (2H, ABq. *J* 12Hz), 5.42 (1H, s), 5.45 (1H, dd, *J* 5.5 and 9Hz), 5.91 (1H, dd, *J* 8 and 16Hz), 6.68 (1H, d, *J* 16Hz), and 6.7—7.5 (11H, m). (found: C, 69.4; H, 5.5; N, 5.7. $C_{28}H_{26}N_2O_6$ requires C, 69.1; H, 5.3; N, 5.8%).

Example 16

*cis* 1-[1-Benzyloxycarbonyl-2-methoxycarbonyl-2-triphenylphosphoranylidene ethyl]-3-phenoxyacetamido-4-styrylazetidin-2-one (26)

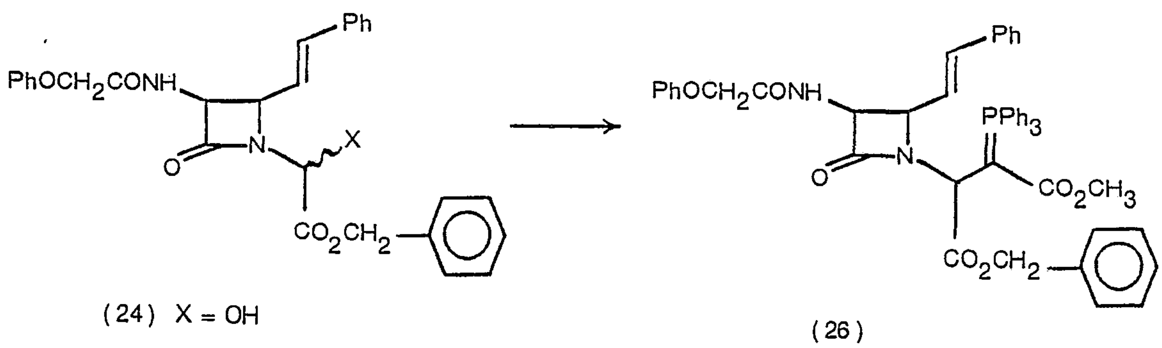

(24) X = OH

(25) X = Cl

(26)

The hydroxy-compound (24) (0.972 g) was dissolved in dry tetrahydrofuran (20 ml) and cooled to −20°C. 2,6-Lutidine (0.322 g) was added, followed by dropwise addition of thionyl chloride (0.350 g) in dry tetrahydrofuran (2 ml) during 10 min. After 5 min. the mixture was diluted with dry toluene, filtered, and the filtrate was evaporated and dried *in vacuo* for 15 min. to give (25) as an oil. The total crude product (25) was dissolved in dry methylene dichloride (30 ml) containing carbomethoxymethylenetriphenylphosphorane (1.51 g). The solution was stirred at room temperature under argon for 24h, then evaporated and the residue chromatographed on silica H to give the product (26) (1.35 g) as an inseparable mixture of isomers $\nu_{max.}$ (Nujol) 3330, 1765, 1760, 1732, 1690, 1685 and 1638 $cm^{-1}$; (Found: C, 72.8; H, 5.4; N, 3.5. $C_{49}H_{43}N_2O_7P$ requires C, 73.3; H, 5.4; N, 3.5%).

Example 17

(2RS, 5RS, 6SR) Benzyl 1-Aza-3-methoxycarbonyl-6-phenoxyacetamidobicyclo-[3.2.0]-hept-3-en-7-one-2-carboxylate (14) and cis 1-[(IRS)-1-Benzyloxycarbonyl-2-methoxycarbonyl-2-triphenylphosphoranylidene ethyl]-4-formyl-3-phenoxyacetamidoazetidin-2-one (15)

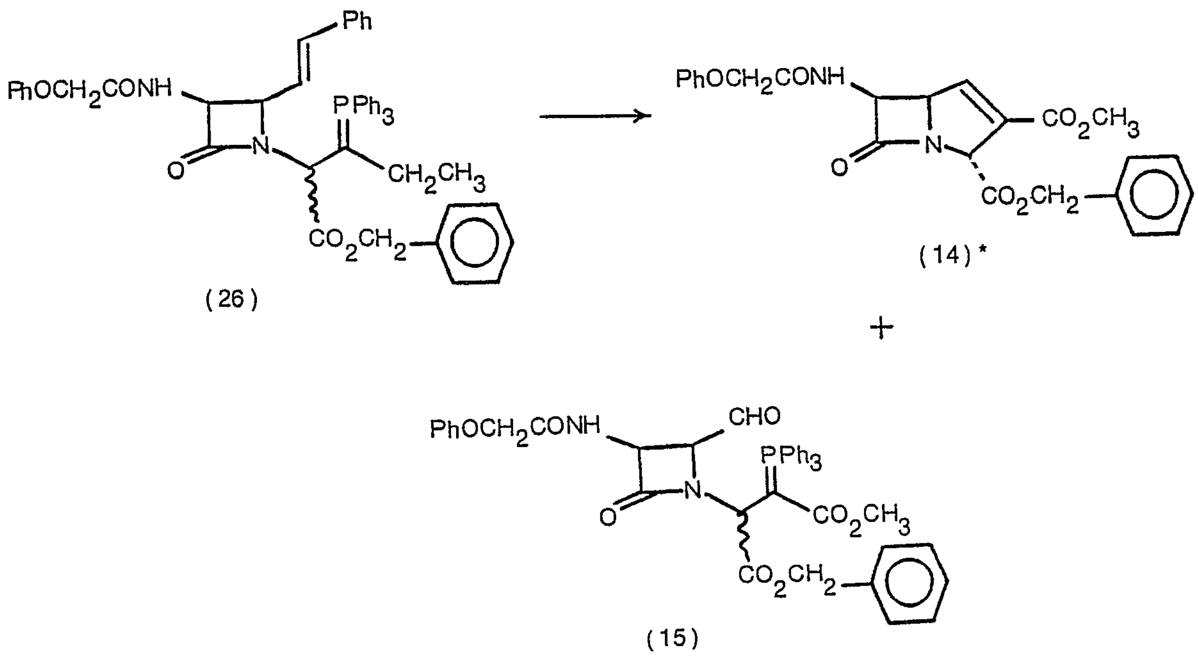

(26)

(14)*

+

(15)

Trifluoroacetic acid (2.5 ml) was added to the β-lactam (26) (847 mg) in ethyl acetate (25 ml), and after 10 min. the solution was cooled to −76° and ozonised oxygen bubbled through until a pale blue colour persisted. Argon was passed through the solution for 20 min. followed by the addition of triphenylphosphine (275 mg) in ethyl acetate (1 ml), pre-cooled to −76°. After 45 min. the reaction mixture was transferred to an icebath and carefully neutralised with saturated aqueous $NaHCO_3$. The organic phase was separated, washed with brine (× 3), dried ($MgSO_4$) and evaporated to an oil. Chromatography on silica H provided the products (14) (200 mg) and (15) (289 mg) (contaminated with some triphenylphosphine oxide) which were identical to those obtained in Example 8.

**Claims**

1. A compound of formula (I):

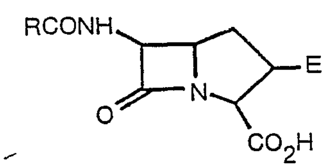

(I)

or a pharmaceutically acceptable salt or cleavable ester thereof, wherein RCONH— represents an organic acylamino group found in antibacterially effective penicillins and cephalosporins and E is a cyano group or a carboxy group or a pharmaceutically acceptable salt or ester thereof of sub-formula (L) or (M):

$$CO_2B_1 \quad (L)$$

$$CO_2CHB_2B_3 \quad (M)$$

wherein $B_1$ is an alkyl group of 1 to 6 carbon atoms optionally substituted by an alkoxy or alkanoyloxy group of 1 to 7 carbon atoms, $B_2$ is an alkenyl or alkynyl group of up to 5 carbon atoms or is a phenyl group optionally substituted by one, two or three atoms or groups selected from fluorine, chlorine, bromine, nitro or alkyl or alkoxy of up to 4 carbon atoms; and $B_3$ is a hydrogen atom, an alkyl group of up to 4 carbon atoms or is a phenyl group optionally substituted by one, two or three atoms or groups selected from fluorine, chlorine, bromine, nitro or alkyl or alkoxy of up to 4 carbon atoms.

2. A compound as claimed in claim 1 wherein E is carboxy or a group of sub-formula (L) wherein $B_1$ is an alkyl of 1 to 6 carbon atoms.

3. A compound as claimed in claim 1 or claim 2 wherein the group RCONH— represents a group of formula (e) or (f):

$$R^2\text{—}\underset{\substack{|\\ R^3}}{CH}\text{—CO—NH} \qquad (e)$$

$$R^4\text{—}\underset{\substack{|\\ R^5}}{CH}\text{—CO—NH} \qquad (f)$$

wherein $R^2$ is a phenyl, thienyl or phenoxy group; $R^3$ is a hydrogren atom or methyl group; $R^4$ is a phenyl, p-hydroxyphenyl, cyclohexadienyl, or a 5- or 6-membered heteroaryl or heterocyclyl group containing up to 3 hetero atoms selected from sulphur, oxygen and nitrogen, said group being optionally substituted by one two or three substituents selected from hydroxy, amino, halo and $C_{1-6}$ alkoxy; $R^5$ is hydroxy, amino or carboxy or phenyl, methylphenyl, indanyl, or $C_{1-6}$ alkyl ester thereof, or ureido of sub formula —$NHCONR^7R^8$ wherein $R^7$ is hydrogen or $C_{1-6}$ alkyl and $R^8$ is $C_{1-6}$ alkyl or an optionally substituted 5- or 6-membered heteroaryl or heterocyclyl group containing one or two nitrogen atoms, or $R^7$ and $R^8$ together with the nitrogen atom to which they are joined form an optionally substituted heteroacyl or heterocyclyl ring containing 1 or 2 nitrogen atoms, wherein the optional substituents for $R^8$ and for the rings formed by $R^7$ and $R^8$ together are one, two or three groups selected from $C_{1-6}$ alkyl, $C_{2-6}$ alkenyl, $C_{2-6}$ alkynyl, $C_{3-6}$ cycloalkyl, phenyl, oxo, hydroxy, $C_{1-6}$ alkoxy, $C_{2-6}$ alkenyloxy, $C_{3-6}$ cycloalkyloxy, phenoxy, mercapto, phenylthio, $C_{1-6}$ alkylthio, amino, $C_{1-6}$ alkylamino, phenylamino, benzylamino, sulphonylamino, $C_{1-6}$ alkyl sulphonylamino or p-aminosulphonylphenylamino, or two substituents on the ring $R^8$ or on the ring formed by $R^7$ and $R^8$ together may form the residue of a further carbocyclic or heterocyclic ring.

4. A compound as claimed in claim 3 wherein RCONH— represents phenoxyacetamido or phenylacetamido.

5. A compound as claimed in claim 1 selected from: (2R,5R,6S) 1 - aza - 3 - methoxycarbonyl - 6 - [D,α - (4 - ethyl - 2,3 - dioxopiperazine - 1 - carboxylamino)phenylacetamido] - bicyclo[3.2.0]heptan - 7 - one - 2 - carboxylic acid; (2R,5R,6S) 1 - aza - 3 - methoxycarbonyl - 6 - phenylacetamido - bicyclo[3.2.0]heptan - 7 - one - 2 - carboxylic acid; (2R,5R,6S) 1 - aza - 3 - methoxycarbonyl - 6 - phenoxyacetamido - bicyclo[3.2.0]heptan - 7 - one - 2 - carboxylic acid; (2R,5R,6S) 1 - aza - 3 - methoxycarbonyl - 6 - [D,2 - (2 - (4 - aminosulphonylanilino) - 4 - hydroxypyrimidyl - 5 - aminocarbonylamino) - 2 - (4 - hydroxyphenyl)acetamido]bicyclo[3.2.0]heptan - 7 - one - 2 - carboxylic acid; (2R,5R,6S) 1 - aza - 3 - methoxycarbonyl - 6 - [D,2 - 2 - (4 - aminosulphonylanilino) - 4 - hydroxypyrimidyl - 5 - aminocarbonylamino) - 2 - (phenyl)acetamido]bicyclo[3.2.0]heptan - 7 - one - 2 - carboxylic acid; and pharmaceutically acceptable salts thereof.

6. A process for the preparation of a compound as claimed in claim 1 which process comprises the reduction of a compound of the formula (VII):

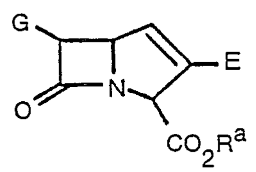

(VII)

wherein $R^a$ is a hydrogen atom or carboxy-blocking group, E is as defined in claim 1 and G is a group RCONH— wherein R is as defined in claim 1 or G is an azido, amino or protected amino group; and thereafter:

i) if G is azido or protected amino converting G to amino;

ii) acylating any compound wherein G is amino with an N-acylating derivative of a carboxylic acid of the formula (VIII):

$$RCO_2H \qquad (VIII)$$

wherein R is as hereinabove defined and any group capable of being acylated is optionally protected;

iii) converting an ester to form an acid or pharmaceutically acceptable salt;

iv) converting an acid or salt to form a cleavable ester.

7. A pharmaceutical composition comprising a pharmaceutically acceptable carrier and a compound as claimed in any one of claims 1 to 5.

8. A compound as claimed in any one of claims 1 to 5 for use as an antibacterial agent.

**Patentansprüche**

1. Eine Verbindung der Formel (I):

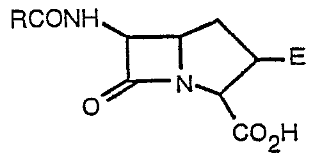

(I)

oder ein pharmazeutisch verträgliches Salz oder ein spaltbarer Ester derselben, wobei die Gruppe RCONH—eine organische Acylaminogruppe darstellt, die in antibakteriell wirkenden Penicillinen und Cephalosporinen vorkommt, und E eine Cyano- oder eine Carboxygruppe oder ein pharmazeutisch verträgliches Salz oder ein Ester derselben mit der Unterformel (L) oder (M) ist:

$$CO_2B_1 \qquad (L)$$

$$CO_2CHB_2B_3 \qquad (M)$$

wobei $B_1$ eine Alkylgruppe mit 1 bis 6 Kohlenstoffatomen, gegebenenfalls substituiert durch eine Alkoxy- oder Alkanoyloxygruppe mit 1 bis 7 Kohlenstoffatomen, ist, $B_2$ eine Alkenyl- oder Alkinylgruppe mit bis zu 5 Kohlenstoffatomen oder eine Phenylgruppe, gegebenenfalls substituiert durch 1,2 oder 3 Atome oder Gruppen ausgewählt aus Fluor, Chlor, Brom, Nitro oder alkyl oder Alkoxy mit bis zu 4 Kohlenstoffatomen, darstellt, und $B_3$ ein Wasserstoffatom, eine Alkylgruppe mit bis zu 4 Kohlenstoffatomen oder eine Phenylgruppe, gegebenenfalls substituiert durch 1, 2 oder 3 Atome oder Gruppen ausgewählt aus Fluor, Chlor, Brom, Nitro oder Alkyl oder Alkoxy mit bis zu 4 Kohlenstoffatomen, darstellt.

2. Eine Verbindung wie in Anspruch 1 beansprucht, in welcher E eine Carboxygruppe oder eine Gruppe der Unterformel (L) ist, wobei $B_1$ eine Alkylgruppe mit 1 bis 6 Kohlenstoffatomen darstellt.

3. Eine Verbindung wie in Anspruch 1 oder 2 beansprucht, in welcher die Gruppe RCONH— eine Gruppe der Formel (e) oder (f) bedeutet:

$$R^2\text{—}\underset{\displaystyle R^3}{\underset{|}{CH}}\text{—CO—NH} \qquad (e)$$

$$R^4\text{—}\underset{\displaystyle R^5}{\underset{|}{CH}}\text{—CO—NH} \qquad (f)$$

wobei $R^2$ eine Phenyl-, Thienyl- oder Phenoxygruppe ist; $R^3$ ein Wasserstoffatom oder eine Methylgruppe ist; $R^4$ eine Phenyl-, p-Hydroxyphenyl-, Cyclohexadienyl- oder eine 5- oder 6-gliedrige Heteroaryl- oder Heterocyclylgruppe mit bis zu 3 Heteroatomen, ausgewählt aus Schwefel, Sauerstoff und Stickstoff, ist, wobei die genannte Gruppe gegebenenfalls substituiert ist durch 1, 2 oder 3 Substituenten ausgewählt aus Hydroxy, Amino, Halogen und $C_{1-6}$-Alkoxy; $R^5$ eine Hydroxy- Amino- oder Carboxygruppe oder eine Phenyl- Methylphenyl-, Indanyl- oder ein $C_{1-6}$-Alkylester derselben, oder eine Ureidogruppe der Unterformel —$NHCONR^7R^8$ wobei $R^7$ Wasserstoff oder eine $C_{1-6}$-Alkylgruppe ist und $R^8$ eine $C_{1-6}$-Alkylgruppe oder eine gegebenenfalls substituierte 5- oder 6-gliedrige Heteroaryl- oder Heterocyclylgruppe mit 1 oder 2 Stickstoffatomen ist, oder $R'$ und $R^8$ zusammen mit dem Stickstoffatom, an das sie gebunden sind, einen gegebenenfalls substituierten Heteroaryl- oder Heterocyclylring mit 1 oder 2 Stickstoffatomen bilden, wobei die möglichen Substituenten für $R^8$ und für die durch $R^7$ und $R^8$ zusammen gebildenten Ringe 1, 2 oder 3 Gruppen ausgewählt aus $C_{1-6}$-Alkyl, $C_{2-6}$-Alkenyl, $C_{2-6}$-Alkinyl, $C_{3-6}$-Cycloalkyl, Phenyl, Oxo, Hydroxy, $C_{3-6}$-Alkoxy, $C_{2-6}$Alkenyloxy, $C_{3-6}$Cycloalkoxy, Phenoxy, Mercapto, Phenylthio, $C_{1-6}$-Alkylthio, Amino, $C_{1-6}$-Alkylamino, Phenylamino, Benzylamino, Sulfonylamino, $C_{1-6}$-Alkylsulfonylamino oder p-Aminosulfonylphenylamino sind, oder 2 Substituenten am Ring $R^8$ oder dem von $R^7$ und $R^8$ zusammen gebildeten Ring den Rest eines weiteren carbozyklischen oder heterozyklischen Rings bilden.

4. Eine Verbindung wie in Anspruch 3 beansprucht, in welcher die Gruppe RCONH— eine Phenoxyacetamido oder Phenylacetamidogruppe darstellt.

5. Eine Verbindung wie in Anspruch 1 beansprucht, ausgewählt aus (2R,5R,6S) 1 - Aza - 3 - methoxycarbonyl - 6 - [D,α - (4 - Äthyl - 2,3 - dioxopiperazin - 1 - carboxylamino)phenylacetamido] - bicyclo[3.2.0]heptan - 7 - on - 2 - carbonsäure; (2R,5R,6S) 1 - Aza - 3 - methoxycarbonyl - 6 - phenylacetamido - bicyclo[3.2.0]heptan - 7 - on - 2 - carbonsäure; (2R,5R,6S) 1 - Aza - 3 - methoxycarbonyl - 6 - phenoxyacetamido - bicyclo[3.2.0]heptan - 7 - on - 2 - carbonsäure; (2R,5R,6S) 1 - Aza - 3 - methoxycarbonyl - 6 - [D,2 - (2 - (4 - aminosulfonylanilino) - 4 - hydroxypyrimidyl - 5 - aminocarbonylamino) - 2 - (4 - hydroxyphenyl)acetamido]bicyclo[3.2.0]heptan - 7 - on - 2 -

carbonsäure; (2R,5R,6S) 1 - Aza - 3 - methoxycarbonyl - 6 - [D,2 - (2 - (4 - aminosulfonylanilin) - 4 - hydroxypyrimidyl - 5 - aminocarbonylamino) - 2 - (phenyl)acetamido]bicyclo[3.2.0]heptan - 7 - on - 2 - carbonsäure und pharmazeutisch verträgliche Salze davon.

6. Ein Verfahren zur Herstellung einer Verbindung wie in Anspruch 1 beansprucht, welches Verfahren die Reduktion einer Verbindung der Formel (VII):

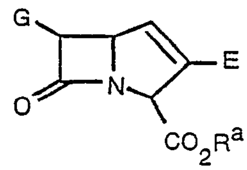

(VII)

in welcher $R^a$ ein Wasserstoffatom oder eine Carboxyblokkierungsgruppe ist, E wie in Anspruch 1 definiert ist und G eine Gruppe RCONH- ist, wobei R wie in Anspruch 1 definiert ist oder G eine Azido, Amino- oder mit einer Schutzgruppe versehene Aminogruppe ist, und anschliessend:

(i) die Umwandlung von G in eine Aminogruppe, wenn G eine Azido oder geschützte Aminogruppe ist;

(ii) die Acylierung irgendeiner Verbindung, in Welcher G eine Aminogruppe darstellt, mit einem V-acylierenden Derivat einer Carbonsäure der Formel (VIII):

$$RCO_2H \qquad (VIII)$$

in welcher R wie oben definiert ist und jede acylierbare Gruppe gegebenenfalls geschützt ist;

(iii) die Umwandlung eines Esters in eine Saüre oder ein pharmazeutisch verträglisches Salz; und

(iv) die Umwandung einer Säure oder eines Salz in einen spaltbaren Ester,

umfaßt.

7. Eine pharmazeutische Zusammensetzung umfassend einen pharmazeutisch verträglichen Träger und eine Verbindung wie in irgendeinem der Ansprüche 1 bis 5 beansprucht.

8. Eine Verbindung wie in irgendeinem der Ansprüche 1 bis 5 beansprucht zur Verwendung als antibakterieller Wirkstoff.

**Revendications**

1. Composé représenté par la formule (I) ci-après:

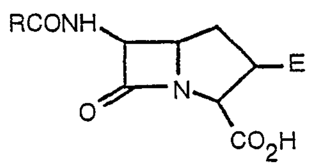

(I)

ou un sel acceptable du point de vue pharmaceutique ou un ester détachable de celui-ci, dans laquelle RCONH est un groupe acylamino organique trouvé dans des pénicillins et céphalosporines efficaces du point de vue antibactérien et E est un groupe cyano ou carboxy ou un sel acceptable du point de vue pharmaceutique ou un ester de celui-ci ayant la sous-formule (L) ou (M):

$$CO_2B_1 \qquad (L)$$

$$CO_2CHB_2B_3 \qquad (M)$$

où $B_1$ est un groupe alcoyle de 1 à 6 atomes de carbone éventuellement substitué par un groupe alcoxy ou alcanoyloxy de 1 à 7 atomes de carbone, $B_2$ est un groupe alcényle ou alcynyle ayant jusqu'à 5 atomes de carbone ou un groupe phényle éventuellement substitué par un, deux ou trois atomes chosis parmi les atomes de fluor, de chlore, de brome ou groupes choisis parmi les radicaux nitro, alcoyle ou alcoxy ayant jusqu'à 4 atomes de carbone; et $B_3$ est un atome d'hydrogène, un groupe alcoyle ayant jusqu'à 4 atomes de carbone ou un groupe phényle éventuellement substitué par un, deux ou trois atomes de fluor, de chlor ou de brome, ou groupes nitro, alcoyle ou alcoxy ayant jusqu'à 4 atomes de carbone.

2. Composé suivant la revendication 1, caractérisé en ce que E est un groupe carboxy ou un groupe de sous-formule (L), où $B_1$ est un radicale alcoyle de 1 à 6 atomes de carbones.

3. Composé suivant la revendication 1 ou 2, caractefisé en ce que le groupe RCONH represénte un groupe de formule (e) our (f):

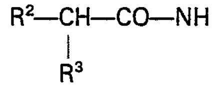

(e)

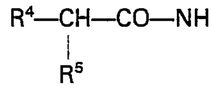

(f)

où $R^2$ est un groupe phènyle, thiényle ou phénoxy; $R^3$ est un atome d'hydrogène ou un groupe méthyle; $R^4$ est un groupe phényle, p-hydroxyphényle, cyclohexadiènyle, ou un groupe hétéroaryle à 5 ou 6 chaînons ou hétérocyclyle contenant jusqu'à 3 hétéroatomes choisis parmi les atomes de soufre, d'oxygène et d'azote, ce groupe étant éventuellement substitué par un, deux ou trois substituants choisis parmi les radicaux hydroxy, amino, halo et alcoxy $C_{1-6}$; $R^5$ est un groupe hydroxy, amino ou carboxy ou un ester phénylique, méthylphénylique, indanylique ou alcoylique en $C_{1-6}$ de celui-ci, ou uréido de sous formule —$NHCONR^7R^8$, dans laquelle $R^7$ est un atome d'hydrogène ou un groupe alcoyle en $C_{1-6}$ et $R^8$ est un groupe alcoyle en $C_{1-6}$ ou un groupe éventuellement substitué hétéroaryle à 5 ou 6 chaînons ou hétérocyclyle contenant un ou deux atomes d'azote, ou bien $R^7$ et $R^8$ forment avec l'atome d'azote auquel ils sont réunis un noyau éventuellement substitué hétéroaryle ou hétérocyclyle contenant 1 ou 2 atomes d'azote, où les substituants éventuels pour $R^8$ et pour les cycles formés par $R^7$ et $R^8$ pris ensembles, comprennent un, deux ou trois groupes choisis parmi les groupes alcoyle en $C_{1-6}$, alcényle en $C_{1-6}$, alcynyle en $C_{1-6}$, cycloalcoyle en $C_{3-6}$, phényle, oxo, hydroxy, alcoxy en $C_{1-6}$, alcényloxy en $C_{2-6}$, cycloalcoyloxy en $C_{3-6}$, phénoxy, mercapto, phénylthio, alcoylthio en $C_{1-6}$, amino, alcoylamino en $C_{1-6}$, phénylamino, benzylamino, sulfonylamino, alcoyl($C_{1-6}$)sulfonylamino ou p-aminosulfonylphénylamino, ou bien deux substituants sur le cycle $R^8$ ou sur le cycle formé par $R^7$ et $R^8$ ensembles, peuvent former le résidu d'un autre cycle carbocyclique ou hétérocyclique.

4. Composé suivant la revendication 3, caractérisé en ce que le radical RCONH— représente les groupes phénoxyacétamido ou phénylacétamido.

5. Composé suivant la revendication 1, caractérisé en ce qu'il est choisi parmi: l'acide (2R,5R,6S) 1 - aza - 3 - méthoxycarbonyl - 6 - [D,α - 4 - éthyl - 2,3 - dioxopipérazine - 1 - carboxylamino)-phénylacétamido] - bicyclo[3,2,0]heptan - 7 - one - 2 - carboxylique, l'acide (2R,5R,6S) 1 - aza - 3 - méthoxycarbonyl - 6 - phénylacetamido - bicyclo[3,2,0]heptan - 7 - one - 2 - carboxylique, l'acide (2R,5R,6S) 1 - aza - 3 - méthoxycarbonyl - 6 - phénoxyacét amido - bicyclo[3,2,0]heptan - 7 - one - 2 - carboxylique, l'acide (2R,5R,6S) 1 - aza - 3 - méthoxycarbonyl - 6 - [D,2 - (2 - (4 - aminosulfonylanilino) - 4 - hydroxypyrimidyl - 5 - aminocarbonylamino) - 2 - (4 - hydroxyphényl)-acétamido] - bicyclo[3,2,0]heptan - 7 - one - 2 - carboxylique, l'acide (2R,5R,6S) 1 - aza - 3 - méthoxycarbonyl - 6 - [D,2 - (2 -(4 - aminosulfonylanilino) - 4 - hydroxypyrimidyl - 5 - aminocarbonylamino) - 2 - (phényl)acétamido] - bicyclo[3,2,0]heptan - 7 - one - 2 - carboxylique, et leurs sels acceptables du point de vue pharmaceutique.

6. Procédé de préparation d'un composé suivant la revendication 1, caractérisé en ce qu'il comprend la réduction d'un composé de formule (VII) ci-après:

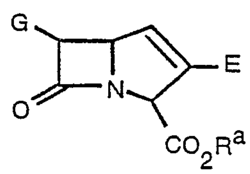

(VII)

dans laquelle $R^a$ est un atome d'hydrogène ou un groupe bloquant le radical carboxy, E est tel que défini dans la revendication 1 et G est un groupe RCONH—, où R est tel que défini dans la revendication 1 ou G est un groupe azido, amino ou protégé, et ensuite,

(i) la conversion de G en grope amino, si G est un groupe azido ou amino protégé;

(ii) l'acylation d'un quelconque composé dans lequel G est un groupe amino, avec un dérivé N-acylant d'un acide carboxylique de formule (VIII) ci-après:

$$RCO_2H \qquad (VIII)$$

dans laquelle R est tel que défini plus haut et un quelconque groupe capable d'être acylé est éventuellement protégé;

(iii) la conversion d'un ester pour former un acide ou un sel acceptable du point de vue pharmaceutique;

(iv) la conversion d'un acide ou d'un sel pour former un ester détachable.

7. Composition pharmaceutique caractérisée en ce qu'elle comprend un support acceptable pharmaceutiquement et un composé suivant l'une quelconque des revendications 1 à 5.

8. Composé suivant l'une quelconque des revendications 1 à 5, caractérisé en ce qu'il est utile comme agent antibactérien.